# EUROPEAN PATENT SPECIFICATION

(11) **EP 4 038 040 B1**
(45) Date of publication and mention of the grant of the patent: **18.11.2026**
(21) Application number: 20871829.6
(22) Date of filing: 29.09.2020
(51) Int. Cl.: C07C 1/24, C07C 11/04, C07C 11/06, C07C 11/10, C07C 11/08, C07C 6/04, C07C 2/12, C10G 3/00, B01J 21/06, B01J 23/30, B01J 29/40, B01J 37/04

(54) **SIMULTANEOUS DEHYDRATION, DIMERIZATION, AND METATHESIS OF C2-C5 ALCOHOLS**
GLEICHZEITIGE DEHYDRATISIERUNG, DIMERISIERUNG UND METATHESE VON C2-C5-ALKOHOLEN
DÉSHYDRATATION, DIMÉRISATION ET MÉTATHÈSE SIMULTANÉES D'ALCOOLS EN C2-C5

(30) Priority: 30.09.2019 US 201962908457 P; 04.10.2019 US 201962910948 P; 14.10.2019 US 201962914837 P; 20.11.2019 US 201962938172 P
(43) Date of publication of application: 10.08.2022
(73) Proprietor: GEVO, Inc., Englewood, CO 80112 (US)
(72) Inventor: SMITH, Jonathan, Englewood, CO 80112 (US); SJODIN, Madeline, Englewood, CO 80112 (US)
(74) Representative: Thum, Bernhard
(86) International application number: PCT/US2020/053312
(87) International publication number: WO 2021/067294

(56) References cited:
- EP-B1- 2 547 639
- US-A- 4 590 320
- US-B1- 7 371 916
- US-B2- 8 450 543
- SADEGHPOUR PARISA, HAGHIGHI MOHAMMAD, KHALEDI KHARAZM: "High-temperature efficient isomorphous substitution of boron into ZSM-5 nanostructure for selective and stable production of ethylene and propylene from methanol'' abstract; page 145, column 1, second paragraph", MATERIALS CHEMISTRY AND PHYSICS, vol. 217, 15 September 2018 (2018-09-15), pages 133 - 150, XP055814235, DOI: 10.1016/j.matchemphys . 2018.06.04 8

## Description

### CROSS-REFERENCE TO RELATED APPLICATIONS

This application claims priority to and the benefit of U.S. Provisional Patent Application Serial No. 62/908,457, filed on September 30, 2019, entitled "Simultaneous Dehydration, Dimerization, Metathesis, and Oligomerization of C2-C5 Alcohols"; U.S. Provisional Patent Application Serial No. 62/910,948, filed on October 4, 2019, entitled "Simultaneous Dehydration, Dimerization, Metathesis, and Oligomerization of C2-C5 Alcohols"; U.S. Provisional Patent Application Serial No. 62/914,837, filed on October 14, 2019, entitled "Simultaneous Dehydration, Dimerization, Metathesis, and Oligomerization of C2-C5 Alcohols"; and U.S. Provisional Patent Application Serial No. 62/938,172, filed on November 20, 2019, entitled "Simultaneous Dehydration, Dimerization, Metathesis, and Oligomerization of C2-C5 Alcohols.

### TECHNICAL FIELD

The subject matter described herein relates to a process for converting one or more C₂-C₅ linear or branched alcohols to one or more C₂-C₇ olefins. The one or more C₂-C₅ alcohols can be from bio-based processes. The process can be performed with two reactors in series, or in one reactor with two catalyst beds. The C₂-C₇ olefins are suitable for oligomerization and conversion into gasoline, jet, and/or diesel fuels.

### BACKGROUND

There is an increasing demand for the use of biomass sources for replacing petroleum as the starting point for the synthesis of fuels. The use of biomass-derived alcohols for the synthesis of base stocks for fuels is therefore of great interest. The reactions for converting alcohols to a base stock for fuels typically start with dehydration followed by olefin oligomerization. C₂-C₅ alcohols are traditionally dehydrated in a single unit operation, at between 300-500 °C in the presence of a dehydration catalyst, resulting in production of the C₂-C₅ olefin along with water. The water is removed, and the C₂-C₅ olefin is further processed/purified to remove unreacted C₂-C₅ alcohols and/or impurities prior to conversion to chemicals and/or fuels. Relative to ethanol (C₂ alcohol), the traditional approach to conversion to chemicals and/or fuels utilizes discrete unit operations to accomplish i) dehydration to ethylene, ii) ethylene purification followed by dimerization to butenes, iii) metathesis to propylene, iv) oligomerization of butenes to unsaturated Jet and/or Diesel fuel precursors, or v) direct oligomerization of ethylene to unsaturated Jet and/or Diesel fuel precursors. Similarly, the approach to converting C₄ or C₅ alcohols to chemicals and/or fuels utilize discrete unit operations to accomplish i) dehydration to the C₄ or C₅ olefin, ii) olefin purification to remove oxygenates and/or unreacted alcohols, and iii) oligomerization to unsaturated Jet and/or Diesel fuel precursors. Efforts have been made to convert alcohols into fuel stocks as described below.

International patent application WO2010/097175A1 relates to the direct conversion of oxygenated compounds to liquid hydrocarbons with a low aromatic content. The process may be carried out via a two-stage process in which both the first and second stage reactors in series both reactors use zeolite catalysts. Methanol single pass conversion is typically > 95 % and the reported final liquid product mainly consists of 60-85 % Cₙ olefins with n ≥ 5, between 15-40 % by weight of light Cₙ olefins with n=2-4, and < 10 % aromatics. The final liquid product is hydrogenated to give gasoline cuts, or oligomerized according to conventional processes to give mixtures of gasoline, kerosene, and diesel. However, the examples describe methanol, which is more toxic than ethanol, and no examples are given with ethanol as feed. Further, the major products are higher carbon olefins (n > 5) instead of light carbon (2 < n < 4) olefins, which may be the result of using zeolite type catalysts in both reactors.

U.S. Pat. No. 8,552,241 relates to a method for converting ethanol in a single step to a diesel fuel base stock which comprises contacting ethanol with an acid catalyst at a reaction temperature of 300-500 °C. The catalyst used is a 50/50 mixture of a γ-alumina in combination with a commercial Axens catalyst 'type IS463' marketed as an alumina-based catalyst for skeletal isomerization of C₄ and C₅ olefin cuts. Only one reactor and only one catalyst type is used. The typical single pass product distribution consisted of a hydrocarbon fraction of 40-50 %, and an organic liquid phase yield of 5-20 %. The organic liquid phase consists of ~50 % olefins of which C₆ olefins are the majority, and ~40 % has a boiling point above 150 °C, and therefore compatible with the diesel pool. The 40-50 % hydrocarbon gaseous phase predominately contains ethylene and ethane as well as traces of C₁, C₃, C₄ and C₅. In this case, the yield to the organic liquid phase is relatively low with ~20 % having a boiling point above 150 °C. The other 80 % of the organic liquid and hydrocarbon fraction is a predominately ethylene and ethane as well as traces of C₁, C₃, C₄ and C₅ and C₆ olefins. Thus, the process described are not efficient or economical.

U.S. Pat. No. 9,840,676 relates to a method for converting ethanol in a three-step process into fuels which can be utilized as full performance or military jet or diesel fuels. The process begins with ethylene formation followed by trimerization to form hexenes and finally oligomerization to jet and diesel fractions. The multistage process converts ethanol into lower carbon olefins, such as C₂-C₄ olefins, then requires further oligomerization to produce fuels such as gasoline and diesel. The catalysts used in trimerization of ethylene to hexenes were Ziegler Natta catalysts.

EP2547639 B1 describes the production of propylene via simultaneous dehydration and skeletal isomerisation of isobutanol which can be obtained by fermentation of biomass. In this process, isobutanol is introduced in a reactor and contacted with a catalyst which can be silicated, zirconated or titanated alumina and the recovered butene stream is sent to a metathesis reactor to produce propylene.

In traditional ethanol dehydration processes, ethanol conversion is nearly complete. The increase of C₂-selectivity while keeping a high ethanol conversion is of importance to gain in process efficiency and to save expensive steps of downstream separation/purification. The reaction products are then mainly water and ethylene. The ethylene being obtained with selectivity as high as 96 %. The subsequent oligomerization of ethylene requires high gauge pressures, generally ranging between 2-4 MPa, but lower temperatures, generally between 20-200 °C. However, the direct oligomerization of ethylene results in relatively low amounts (~40 % highest reported level) of C₁₀₊ or diesel fraction.

### SUMMARY

Aspects of the current subject matter relate to processes for converting C₂-C₅ alcohols to C₂-C₇ olefins, as defined by the appended claims.

Consistent with some aspects of the current subject matter, a processes for converting one or more C₂-C₅ linear or branched alcohols to one or more C₂-C₅ olefins is disclosed. The process includes two different catalyst systems either using either two reactors in series wherein one catalyst is in each reactor, or one reactor with two catalyst beds. For processes which use two reactors in series, the water product from the first reactor can be separated from the stream before entering the second reactor, or left in the stream.

In variations, one or more of the following features may be included in any feasible combination. For example, the one or more C₂-C₅ linear or branched alcohols can be C₂-C₄ linear or branched alcohols. The one or more C₂-C₅ olefins formed by the processes described herein can be C₂-C₆ olefins. Further, the mass yield of the one or more C₂-C₇ olefins can be between 70 % to 99 %.

The temperature, pressure, and WHSV of processes which occur in two reactors in series can be different between the two reactors, or the same. For example, the temperature of the first reactor can be from 300 °C to 450 °C including all the subranges in between, and the temperature of the second reactor can be from 320 °C to 450 °C including all the subranges in between. The gauge pressure of the first reactor and second reactors can independently be from 0 to 10 bar including all the subranges in between. The WHSV of the first reactor and second reactor are each independently from 0.5 to 10 and all the subranges in between.

In processes which occur in one reactor with two catalyst beds, the temperature, pressure, and WHSV are consistent as the feed stream passes from the first catalyst bed to the second catalyst bed. The temperature of the one reactor processes can be from 300 °C to 400 °C including all the subranges in between. The pressure can be from 0 to 10 bar including all subranges in between. The WHSV can be from 0.5 to 10 and all the subranges in between.

The processes described herein comprise two catalyst systems regardless of whether the reaction is performed in one or two reactors. The first catalyst comprises γ-alumina catalyst, which can comprise Zr. The first catalyst may not comprise a zeolite catalyst. The second catalyst comprises zeolite catalyst which comprises boron, phosphorus, or both. The second catalyst can also include γ-alumina catalyst, W-SiO₂ catalyst, or W-Zr catalyst, wherein the γ-alumina optionally includes Zr, W, or Mo.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter of this disclosure are contemplated as being part of the inventive subject matter disclosed herein.

### DETAILED DESCRIPTION

In the following description, certain specific details are set forth in order to provide a thorough understanding of various embodiments. However, one skilled in the art will understand that the disclosure may be practiced without these details. In other instances, well-known structures have not been shown or described in detail to avoid unnecessarily obscuring descriptions of the embodiments. Unless the context requires otherwise, throughout the specification and claims which follow, the word "comprise" and variations thereof, such as, "comprises" and "comprising" are to be construed in an open, inclusive sense, that is, as "including, but not limited to." Further, headings provided herein are for convenience only and do not interpret the scope or meaning of the claimed disclosure.

Reference throughout this specification to "one embodiment" or "an embodiment" means a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments. Also, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

The word "about" when immediately preceding a numerical value means a range of plus or minus 10 % of that value, e.g., "about 50" means 45 to 55, "about 25,000" means 22,500 to 27,500, etc. Furthermore, the phrases "less than about" a value or "greater than about" a value should be understood in view of the definition of the term "about" provided herein.

"Oxygenate" refers to compounds which include oxygen in their chemical structure. Examples of oxygenates include, but are not limited to water, alcohols, esters, and ethers.

"WHSV" refers to weight hourly space velocity and is defined as the weight of the feed flowing per unit weight of the catalyst per hour.

"Single stage transformation" refers to processes which occur within a single reactor system.

All yields and conversions described herein are on a mass basis unless specified otherwise.

Described herein are processes to convert alcohols in a single-stage reactor with dual catalysts, or in a two-stage reactor configuration (in series) with separate catalyst beds. The alcohols are converted to an olefinic mixture comprised of primarily C₃-C₇ olefins with low levels of aromatic compounds. The processes provide paths towards economical ways to convert alcohols, e.g. ethanol, to base stocks for the production of fuels. Further, the processes described herein can be performed at lower pressures and higher temperatures with higher yields of olefins in comparison to previous reports. The processes comprise a single stage transformation of an aqueous bioalcohol feedstock derived from biomass into a higher molecular weight olefinic mixture, which can easily be oligomerized in high yield to C₁₀₊ hydrocarbons or diesel fractions. The single-stage reactor or two-stage reactor configurations use specific catalytic systems, which make it possible to minimize the production of aromatic compounds and therefore maximize production of middle distillates. This constitutes both an asset for the ethanol refiner and an advantage from the standpoint of lasting development. Processes described herein convert C₂-C₅ alcohols efficiently and economically as a base stock for fuels. Conversion of C₂-C₅ alcohols to the desired fuel product precursors (i.e. C₃-C₇ olefins) in high yields reduces processing costs.

Simultaneously dehydrating/dimerizing/metathesizing a C₂-C₅ alcohol in one reactor is challenging due to higher temperatures required for complete dehydration (300-500 °C), and large amounts of water present. Implementation of a single unit operation capable of simultaneously dehydrating, dimerizing, and metathesizing an alcohol requires that catalysts employed be able to withstand high temperatures along with large amounts of water and other oxygenates. The ability to accomplish numerous unit operations and chemical transformations in a single stage provides favorable economics due to reduced fixed and variable costs, lower capital investment, and increased productivity.

Aspects of the currently subject matter overcome challenges by providing processes in which a two-catalyst system is used to convert C₂-C₅ linear or branched alcohols to C₂-C₇ olefins in high yield with low levels of aromatics at competitive costs. The C₂-C₇ olefins can be easily oligomerized to base stocks used in the production of fuels in high yields. The process can be carried out in two catalytic fixed bed reactors in series wherein the first reactor operates at a lower or higher temperature than the operating temperature of the second reactor. The process is comprised of the steps of alcohol dehydration to lower carbon chain olefins and water in high yields using a first catalyst in the first reactor, followed by directly feeding the lower carbon chain olefins and water to the second reactor with conversion of said lower carbon chain olefins and water to a C₂-C₇ olefinic mixture using a second catalyst. Furthermore, in the two-stage process, the water formed during dehydration of alcohols within the first reactor having the first catalyst may be separated from the lower carbon chain olefins after exiting the first reactor prior to feeding into the second reactor including the second catalyst. Alternatively, alcohols, e.g. ethanol, may be converted to olefinic mixtures (C₂-C₇) in a single reactor having the first catalyst in the top section of the reactor with the second catalyst being located in a section of the reactor below the first catalyst. In either the two-stage or single-stage processes, the resulting C₂-C₇ olefinic mixture is suitable for oligomerization into either gasoline, jet, or diesel fuel cuts at relatively low temperatures and pressures depending upon the oligomerization catalyst selected.

One advantage of having the two catalyst beds in two reactors in series, rather than in one reactor, is that it allows for the capability and flexibility to operate the reactors at two different reaction temperatures. In this process, the effluent from the first dehydration reactor, with water vapor and other minor impurities, is directed 'as-is' to the second reactor in series at an optimum temperature depending upon the target fuel and/or olefinic mixture. For example, dehydration of ethanol to ethylene at 360 °C, over silicated, zirconated, titanated, niobium, or fluorinated γ-alumina in the first reactor (fixed bed 'A'), followed by directing the gaseous non-condensed ethylene enriched stream including water and impurities to the second reactor (fixed bed 'B') packed with i) a metathesis catalyst (i.e. tungstated alumina, etc.) admixed with a ZSM-5 zeolite or doped ZSM-5 zeolite, or ii) a doped ZSM-5 zeolite admixed with a zirconated alumina catalyst at an internal temperature between 300-500 °C and at pressures between 0-30 bar and WHSV 0.5-10, accomplishes ethylene dimerization and metathesis to C₃-C₇ olefins in high yields. This two-reactor in series approach enables the practitioner to optimize reactor temperatures independently and allows catalyst regeneration of the second reactor (fixed bed 'B') without disrupting operation of the first reactor (fixed bed 'A').

Disclosed herein is a process for converting one or more C₂-C₅ linear or branched alcohols to one or more C₂-C₇ olefins, the process comprising contacting a first stream comprising the C₂-C₅ linear or branched alcohols with a first catalyst in a first reactor at a temperature from 200 °C to 500 °C, a gauge pressure from 0 to 30 bar, and a WHSV of at least 0.5, wherein the first catalyst comprises an alumina catalyst including one or more of Si, Zr, Ti, Nb, W or F in neutral or ionic form, to form a second stream; and contacting the second stream with a second catalyst in a second reactor at a temperature from 200 °C to 500 °C, gauge pressure from 0 to 30 bar, and a WHSV of at least 0.5, wherein the second catalyst comprises a doped or undoped zeolite catalyst and/or a doped or undoped W-Zr catalyst and forming the one or more C₂-C₇ olefins in a mass yield of at least 65 %.

An alternative two-reactor scenario can be advantageous for conversion of ethanol into C₃-C₇ olefins in high yield. For example, dehydration of ethanol to ethylene at 360 °C, over silicated, zirconated, titanated, niobium, or fluorinated γ-alumina in the first reactor (fixed bed 'A') followed by water removal and directing the gaseous non-condensed ethylene enriched stream, saturated with water, to the second reactor (fixed bed 'B') packed with i) a metathesis catalyst (i.e. tungstated alumina, etc.) admixed with a ZSM-5 zeolite or doped ZSM-5 zeolite, or ii) a doped ZSM-5 zeolite admixed with a zirconated alumina catalyst at an internal temperature between 300-500 °C and at gauge pressures between 0-30 bar and WHSV 0.5-10 accomplishes ethylene dimerization and metathesis to C3-C7 olefins in high yield. This two-reactor in series approach enables the practitioner to optimize reactor temperatures independently, allows catalyst regeneration of the second reactor (fixed bed 'B') without disrupting operation of the first reactor (fixed bed 'A'), and enables the use of catalyst combinations that exhibit less stability to the presence of large amounts of water.

The present disclosure also provides a process for converting one or more C₂-C₅ linear or branched alcohols to one or more C₂-C₇ olefins, the process comprising: contacting a first stream comprising the C₂-C₅ linear or branched alcohols with a first catalyst in a first reactor at a temperature from 200 °C to 500 °C, a gauge pressure from 0 to 30 bar, and a WHSV of at least 0.5, wherein the first catalyst comprises an alumina catalyst including one or more of Si, Zr, Ti, Nb, W or F in neutral or ionic form, to form a second stream; separating a portion of water from the second stream; and contacting the second stream with a second catalyst in a second reactor at a temperature from 200 °C to 500 °C, a gauge pressure from 0 to 30 bar, and a WHSV of at least 0.5, wherein the second reactor comprises a doped or undoped zeolite catalyst and/or a doped or undoped W-Zr catalyst and forming the one or more C₂-C₇ olefins in a mass yield of at least 70 %.

A single unit operation (i.e. a single reactor), can also be used to convert lower alcohols into a viable feedstock of higher molecular weight olefins in high yield, which can be easily oligomerized to base stocks for fuels in high yield. For example, passing a vaporized stream of undenatured fuel grade (> 92.1 % v/v ethanol/water) or hydrous ethanol over fixed catalyst bed 'A' containing silicated, zirconated, titanated, niobium, or fluorinated γ-alumina at between 300-400 °C at gauge pressures between 0-30 bar and a WHSV between 0.5-10, followed by directly passing the ethylene vapor stream without isolation, purification, or removal of water over fixed catalyst bed 'B' in the same reactor containing; i) a doped or undoped tungstated zirconium catalyst, or ii) a tungstated, molybdenumated, silicated or zirconated alumina admixed with a doped or undoped ZSM-5 zeolite catalyst, at the same temperature and gauge pressures (i.e. 300-400 °C, 0-30 bar) provides the ethanol producer with a C₃-C₇ olefinic mixture in high yields with minimal aromatic content. The C₃-C₇ olefinic mixture can be separated for sale, or after removal of condensed water, oligomerized 'as-is' to primarily jet and/or diesel fuels in high yields.

This disclosure also describes a process for converting one or more C₂-C₅ linear or branched alcohols to one or more C₂-C₇ olefins, the process comprising contacting a first stream comprising the C₂-C₅ linear or branched alcohols with a first catalyst bed in a reactor at a temperature from 200 °C to 500 °C, a gauge pressure from 0 to 30 bar, and a WHSV of at least 0.5, wherein the first catalyst bed comprises an alumina catalyst including one or more of Si, Zr, Ti, Nb, W or F in neutral or ionic form, to form a second stream; and contacting the second stream with a second catalyst bed in the reactor at the same temperature, pressure, and WHSV as the first reactor, wherein the second catalyst bed comprises a doped or undoped zeolite catalyst and/or a doped or undoped W-Zr catalyst and forming the one or more C₂-C₇ olefins in a mass yield of at least 70 %.

The C₂-C₅ linear or branched alcohols useful in the present disclosure includes any C₂-C₅ linear or branched alcohol known by one of skill in the art. For example the C₂-C₅ alcohols can be one or more of ethanol, propanol, iso-propanol, 1-butanol, isobutanol, 2-butanol, tert-butanol, pentanol, 3-methyl-1-butanol, 2-methyl-1-butanol, 2,2-dimethyl-1-propanol, 3-pentanol, 2-pentanol, 3-methyl-2-butanol, or 2-methyl-2-butanol. The C₂-C₅ alcohols of the present invention can be from bio-based processes, such as, but not limited to, fermentation. For example, the C₂-C₅ linear or branched alcohols are bio-based and produced by fermentative processes. The C₂-C₅ linear or branched alcohols are not derived from petroleum.

Consistent with some aspects of the subject matter, the one or more C₂-C₅ linear or branched alcohols is ethanol, propanol, isopropanol, 1-butanol, isobutanol, 2-butanol, or tert-butanol. In many instances, the one or more C₂-C₅ linear or branched alcohols is ethanol, propanol, butanol, or a combination thereof. For example, the one or more C₂-C₅ linear or branched alcohols is ethanol. In another example, the one or more C₂-C₅ linear or branched alcohols is isobutanol.

Consistent with some aspects of the subject matter, the one or more C₂-C₇ olefins of any processes described herein are any suitable olefin. In many instances the one or more C₂-C₇ olefins is one or more C₂-C₆ olefins. In some examples, the one or more C₂-C₇ olefins includes ethylene, propylene, butenes, C₅-C₆ olefins, or a combination thereof.

The temperature for the processes described herein can be any suitable reactor temperature known by one of skill in the art. In the processes comprising two reactors, the temperature of the first reactor can be higher, lower, or the same as the second reactor. In some instances, the temperature of the first reactor is from 300 °C to 450 °C. In some examples, the temperature of the first reactor is from 300 °C to 420 °C. For example the temperature of the first reactor is about 360 °C.

In some examples, the temperature of the second reactor is from 320 °C to 420 °C. For example the temperature of the second reactor is from 340 °C to 400 °C. In some examples, the temperature of the second reactor is from 350 °C to 450 °C. For example, the temperature of the second reactor is from 380 °C to 410 °C.

In the processes comprising one reactor with two catalyst beds, the temperature of the reactor can be any suitable temperature. The temperature is from 300 °C to 400 °C. For example, the temperature is about from 330 °C to 360 °C.

The gauge pressure for the processes described herein can be any suitable gauge pressure known by one of skill in the art. In the processes comprising two reactors, the gauge pressure of the first reactor can be higher, lower, or the same as the second reactor. In some instances, the gauge pressure of the first reactor and second reactor are each independently from 0 to 30 bar. In some instances the gauge pressure of the first reactor and second reactor are each independently from 0 to 10 bar. For example, the gauge pressure of the first reactor and second reactor are each independently 0 to 1 bar. In some examples, the gauge pressure of the first reactor and second reactor are each independently 0 bar.

The WHSV for the processes described herein can be any suitable WHSV known by one of skill in the art. In the processes comprising two reactors, the WHSV are each independently at least 0.5. In many instances, the WHSV of the first and second reactors are each independently from 0.5 to 10. For example, the WHSV of the first and second reactors are each independently from 0.5 to 5. In some examples, the WHSV of the first and second reactors are each independently at least 1. In some examples, the WHSV of the first and second reactors are each about 2.1.

In the processes comprising one reactor with two catalyst beds, the WHSV can be from 0.5 to 10. In some examples, the WHSV is at least 1. In some examples, the WHSV is from 2.1 to 2.3.

Granular or extruded catalysts are suitable for the reactions even though no specific size and morphology are mandatory. Catalysts with a size greater than 0.1 mm are more suitable, and the size of 0.4-1.0 mm is most suitable for the operability and pressure drop.

The first catalyst comprises an alumina catalyst including Si, Zr, Ti, Nb, W or F (i.e. a doped alumina catalyst) disclosed herein are more stable in the present process as compared to standard commercial grade alumina catalysts. Consistent with some aspects of the subject matter, the first catalyst comprises an alumina catalyst including one or more of Si (silicon), Zr (zirconium), Ti (titanium), Nb (niobium), W (tungsten) or F (fluorine). One or more of Si, Zr, Ti, Nb, W or F can be in an ionic form. In some instances, the alumina catalyst comprises the Zr. The Zr can be zirconium (IV). The first catalyst may not comprise a zeolite catalyst.

Zr can be present in an amount from 2 wt. % to 10 wt. % in the first catalyst. In some examples, Zr is present in an amount of about 4.0 wt. % in the first catalyst. In other examples, Zr is present in an amount of about 5.6 wt. % in the first catalyst.

The alumina catalyst can be regenerated as necessary under suitable conditions for the processes described herein. Consistent with some aspects of the subject matter, the alumina catalyst is regenerated *in-situ* in air. The alumina catalyst can be regenerated at a temperature of 400 °C to 600 °C. For example, the alumina catalyst can be regenerated at 500 °C. Consistent with some aspects of the subject matter, the alumina catalyst is regenerated for 30 minutes to 3 hours. For example, the alumina catalyst is regenerated for 1 to 2 hours.

The second catalyst, can be 'regenerable' catalyst combinations for C₂-C₅ olefin conversion consist of doped zeolites such as crystalline silicates of the group ZSM-5 (MFI or BEA frameworks), CHA, FER, FAU, MWW, MOR, EUO, MFS, ZSM-48, MTT or TON having Si/Al higher than 10, or a dealuminated crystalline silicate of the group ZSM5 (MFI or BEA frameworks), CHA, FER, FAU, MWW, MOR, EUO, MFS, ZSM-48, MTT or TON having Si/Al higher than 10, or a phosphorus and/or boron modified crystalline silicate of the group ZSM-5 (MFI or BEA frameworks), CHA, FER, FAU, MWW, MOR, EUO, MFS, ZSM-48, MTT or TON having Si/Al higher than 10, or molecular sieves of the type silicoaluminophosphate of the group AEL. Additional additives for mixing with the doped zeolites in the second catalyst consist of SiO2, MgO, or metathesis catalysts (i.e. (tungstated or molybdenum doped alumina, zirconia, or SiO2 supports.

The second catalyst comprises a doped or undoped zeolite catalyst and/or a doped or undoped W-Zr catalyst. In many instances, the second catalyst is a doped or undoped zeolite catalyst. For example, the second catalyst can be a doped ZSM-5 zeolite catalyst. The doped ZSM-5 zeolite catalyst can include boron, phosphorus, or both. For example the doped ZSM-5 catalyst can include boron and phosphorus. In another example, the doped ZSM-5 catalyst can include boron. The second catalyst can further comprise γ-alumina catalyst, W-SiO₂ catalyst, or W-Zr catalyst, and wherein the γ-alumina optionally includes Zr, W, or Mo.

Boron and the phosphorus which are present in an amount of from 2 wt. % to 5 wt. % of the second catalyst. In some instances, boron is present in an amount of about 2.5 wt. % and phosphorus is present in an amount of about 3.7 wt. % of the second catalyst. In some examples, boron is present in an amount of from 2 wt. % to 5 wt. % of the second catalyst. For example, boron is present in an amount of 3.5 wt. % to 4.1 wt. % of the second catalyst.

In some instances, the second catalyst comprises an undoped ZSM-5 zeolite catalyst. The second catalyst comprising the undoped ZSM-5 zeolite catalyst can further comprise Mo-Zr catalyst or W-Zr catalyst.

The C₂-C₇ olefins formed from the process described herein have a mass yield of at least 65 %. In many examples, the mass yield is at least 70 %. The mass yield of the one or more C₂-C₇ olefins is from 65 % to 98 %. For example, the mass yield of the one or more C₂-C₇ olefins is from 75 % to 95 %. In some cases, the mass yield of the one or more C₂-C₇ olefins is from 70 % to 99 %. In some examples, the mass yield of the one or more C₂-C₇ olefins is from 80 % to 95 %.

The present disclosure also provides a process for converting ethanol to one or more C₂-C₇ olefins, the process comprising: contacting a first stream comprising the ethanol with a first catalyst in a first reactor at a temperature of about 360 °C, gauge pressure of 0 bar, and a WHSV of about 2.1, wherein the first catalyst comprises a γ-alumina catalyst including Zr, to form a second stream; and contacting the second stream with a second catalyst in a second reactor at a temperature from 340 °C to 400 °C, gauge pressure of 0 bar, and a WHSV of about 2.1, wherein the second catalyst comprises a doped ZSM-5 zeolite catalyst, wherein the doped zeolite catalyst includes boron, phosphorus, or both and forming the one or more C₂-C₇ olefins in a mass yield from 80 % to 95 %.

Also provided is a process for converting ethanol to one or more C₂-C₇ olefins, the process comprising: contacting a first stream comprising the ethanol with a first catalyst in a first reactor at a temperature of about 360 °C, gauge pressure of about 0 bar, and a WHSV of about 2.1, wherein the first catalyst comprises a γ-alumina catalyst including Zr, to form a second stream; separating a portion of water from the second stream; and contacting the second stream with a second catalyst in a second reactor at a temperature from 380 °C to 410 °C, gauge pressure of about 0 bar, and a WHSV of about 2.1, wherein the second reactor comprises a boron doped ZSM-5 zeolite catalyst and forming the one or more C₂-C₇ olefins in a mass yield from 80 % to 95 %.

The present disclosure further provides a process for converting ethanol to one or more C₂-C₇ olefins, the process comprising: contacting a first stream comprising the ethanol with a first catalyst in a reactor at a temperature from 330 °C to 360 °C, gauge pressure of about 0 bar, and a WHSV from 2.1 to 2.3, wherein the first catalyst comprises a γ-alumina catalyst including Zr, to form a second stream; and contacting the second stream with a second catalyst in the reactor at the same temperature, pressure, and WHSV as the first reactor, wherein the second catalyst comprises an undoped ZSM-5 zeolite catalyst and a W-Zr catalyst or Mo-Zr catalyst and forming the one or more C₂-C₇ olefins in a mass yield from 81 % to 99 %.

### EXAMPLES

### REACTOR SET-UP

Ethanol conversion to C₃-C₇ olefins was carried out at 300-500 °C, via fixed bed reactors, containing specified catalyst(s), and flowing preheated (160 °C) vaporized ethanol in a downward flow over the fixed catalyst bed while co-feeding nitrogen at atmospheric pressure or under moderate gauge pressures (i.e. 0-30 bar). The flow rate of ethanol was controlled by Teledyne Model 500D syringe pumps, and the flow rates were adjusted to obtain the targeted olefin WHSV (weight hourly space velocity). The internal reaction temperature was maintained constant via a Lindberg Blue M furnace as manufactured by Thermo-Scientific. Ethanol conversion and selectivity was calculated by analysis of the liquid phase reactor effluent by GC (gas chromatography) for organic and water content, and online GC analysis of non-condensed hydrocarbons (i.e. C₂-C₅ olefins) relative to nitrogen as internal standard. Thus, passing a vaporized stream of fuel grade ethanol over silicated, zirconated, titanated or fluorinated alumina followed by passing the hydrous ethylene over the second fixed bed catalyst at between 350-450 °C provides the ethanol refiner with the ability to produce C₂-C₇ olefins in high yields.

The following representative examples relate to converting fuel grade or hydrous ethanol to primarily propylene and butenes in > 75 % yields along with lesser amounts of C₅₊ olefins and aromatics (BTX - benzene, toluene, xylene) via a single unit operation at > 60 % ethylene conversion along with quantitative ethanol conversion. Unreacted ethylene may be separated and recycled. Ethylene conversion may be increased, by increasing temperature in the second reactor, but higher ethylene conversions in some cases results in higher levels of aromatics which are more economically unfavorable as compared to higher yields to C₃-C₇ olefins which may be converted to renewable diesel fuel. Conversely, ethylene conversion may be decreased (~40 %) resulting in higher selectivity/yield to C₃-C₇ olefins (~90 %), lesser yield to aromatics, and higher levels of ethylene recycle.

### Catalyst Preparation Example 1a

Impregnated Zr-γ-Alumina (nominal Zr metal 5 wt. %) Catalyst preparation: Zr-γ-alumina catalyst was prepared by incipient wetness technique as described. The precursor metal salts (Sigma Aldrich): 2.64g zirconium (IV) oxynitrate hydrate was dissolved in deionized water (14.9 ml). Upon salt dissolution, the solution was added in dropwise fashion to 15g γ-alumina support. The resulting mixed metal oxide was manually mixed to assure complete wetting, and the resulting impregnated catalyst was dried at 160 °C for 1 hr, and afterwards calcined at 500 °C for 4 hrs.

### Catalyst Preparation Example 1b

Impregnated W-γ-Alumina (nominal W metal 15 wt. %) Catalyst Preparation: W-γ-alumina catalyst was prepared by incipient wetness technique as described. The precursor metal salts (Sigma Aldrich): 1.74g ammonium tungstate pentahydrate hydrate was dissolved in deionized water (9.2 ml). Upon salt dissolution, the solution was added in dropwise fashion to 8g γ-alumina support. The poor solubility of ammonium tungstate salt in water required multiple water dissolutions and additions of the tungstate salt to the γ-alumina support to achieve the required tungstate loading. After each water/salt addition, the resulting mixed metal oxide was manually mixed to assure complete wetting after each tungstate salt addition, and the resulting impregnated catalyst was dried at 160 °C for 1 hr, and afterwards calcined at 500 °C for 4 hrs.

### Catalyst Preparation Example 1c

Impregnated boron and phosphor impregnated ZSM-5 zeolite catalyst preparation: boron and phosphor impregnated zeolite catalyst was prepared by incipient wetness technique as described. 0.83g phosphoric acid (85 %) and 0.94g boric acid was dissolved in deionized water (7.2 ml). Upon dissolution, the solution was added in dropwise fashion to 6g ZSM-5 zeolite support (i.e. Zeolyst type CBV-5524 H⁺). The resulting impregnated catalyst was dried at 160 °C for 1 hr, and afterwards calcined at 550 °C for 3 hrs.

### Example 2

Two-Stage reactor configuration without removal of water from ethanol dehydration: Reaction Conditions: T=360 °C in first reactor, T=340 °C in second reactor WHSV = 2.1 (ethanol basis), P=0 bar; Catalysts - zirconated (5.6 wt. %) γ-alumina in first reactor and boron/phosphorus doped ZSM-5 zeolite (2.5 wt. % boron/3.7 wt. % phosphorus) admixed with Zr-alumina (5.6 wt. % Zr) in second reactor.

### Reactor Effluent Composition - Wt. % of Total:

| | |
|---|---|
| Ethylene | 30 |
| Propylene | 13 |
| Butenes | 27 |
| C₅ olefins | 14 |
| C₂-C₄ saturates | 7 |
| Aromatics | 5 |
| C₆₊ olefins | 4 |

Ethylene conversion ~62 %; mass yield to C₃-C₇ olefins based on ethylene conversion ~81 %; mass yield to aromatics based on ethylene conversion ~8 %.

### Example 3

Two-Stage reactor configuration without removal of water from ethanol dehydration: Reaction Conditions: T=360 °C in first reactor, T=370 °C in second reactor WHSV = 2.1 (ethanol basis), P=0 bar; Catalysts - zirconated (5.6 wt. %) γ-alumina in first reactor and boron/phosphorus doped ZSM-5 zeolite (2.5 wt. % boron/3.7 wt. % phosphorus) admixed with γ-alumina in second reactor.

### Reactor Effluent Composition - Wt. % of Total:

| | |
|---|---|
| Ethylene | 24 |
| Propylene | 15 |
| Butenes | 30 |
| C₅ olefins | 11 |
| C₂-C₄ saturates | 9 |
| Aromatics | 7 |
| C₆₊ olefins | 4 |

Ethylene conversion ~70 %; mass yield to C₃-C₇ olefins based on ethylene conversion ~76 %; mass yield to aromatics based on ethylene conversion ~10 %.

### Example 4

Two-Stage reactor configuration without removal of water from ethanol dehydration: Reaction Conditions: T=360 °C in first reactor, T=380 °C in second reactor WHSV = 2.1 (ethanol basis), P=0 bar; Catalysts - zirconated (5.6 wt. %) γ-alumina in first reactor and boron/phosphorus doped ZSM-5 zeolite (2.5 wt. % boron/3.7 wt. % phosphorus) admixed with W-SiO₂ (W~15 wt. %) in second reactor.

### Reactor Effluent Composition - Wt. % of Total:

| | |
|---|---|
| Ethylene | 39 |
| Propylene | 17 |
| Butenes | 24 |
| C₅ olefins | 14 |
| C₂-C₄ saturates | 4 |
| Aromatics | 1 |
| C₆₊ olefins | 1 |

Ethylene conversion ~40 %; mass yield to C₃-C₇ olefins based on ethylene conversion ~90 %; mass yield to aromatics based on ethylene conversion ~4 %.

### Example 5

Two-Stage reactor configuration without removal of water from ethanol dehydration: Reaction Conditions: T=360 °C in first reactor, T=395 °C in second reactor WHSV = 2.1 (ethanol basis), P=0 bar; Catalysts - zirconated (5.6 wt. %) γ-alumina in first reactor and boron/phosphorus doped ZSM-5 zeolite (2.5 wt. % boron/3.7 wt. % phosphorus) in second reactor.

### Reactor Effluent Composition - Wt. % of Total:

| | |
|---|---|
| Ethylene | 22 |
| Propylene | 17 |
| Butenes | 29 |
| C₅ olefins | 16 |
| C₂-C₄ saturates | 8 |
| Aromatics | 6 |
| C₆₊ olefins | 2 |

Ethylene conversion ~70 %; mass yield to C₃-C₇ olefins based on ethylene conversion ~82 %; mass yield to aromatics based on ethylene conversion ~8 %.

### Example 6

Two-Stage reactor configuration with removal of water from ethanol dehydration: Reaction Conditions: T=360 °C in first reactor, T=410 °C in second reactor WHSV = 2.1 (ethanol basis), P=0 bar; Catalysts - zirconated (5.6 wt. %) γ-alumina in first reactor and boron doped ZSM-5 zeolite (4.1 wt. % boron) in second reactor in second reactor.

### Reactor Effluent Composition - Wt. % of Total:

| | |
|---|---|
| Ethylene | 30 |
| Propylene | 19 |
| Butenes | 24 |
| C₅ olefins | 15 |
| C₂-C₄ saturates | 7 |
| Aromatics | 3 |
| C₆₊ olefins | 2 |

Ethylene conversion ~55 %; mass yield to C₃-C₇ olefins based on ethylene conversion ~85 %; mass yield to aromatics based on ethylene conversion ~6 %.

### Example 7

Two-Stage reactor configuration with removal of water from ethanol dehydration: Reaction Conditions: T=360 °C in first reactor, T=410 °C in second reactor WHSV = 2.1 (ethanol basis), P=0 bar; Catalysts - zirconated (5.6 wt. %) γ-alumina in first reactor and boron doped ZSM-5 zeolite (3.7 wt. % boron) admixed with W-zirconium (W ~15 wt. %) in second reactor.

### Reactor Effluent Composition - Wt. % of Total:

| | |
|---|---|
| Ethylene | 32 |
| Propylene | 13 |
| Butenes | 26 |
| C₅ olefins | 18 |
| C₂-C₄ saturates | 6 |
| Aromatics | 3 |
| C₆₊ olefins | 2 |

Ethylene conversion ~60 %; mass yield to C₃-C₇ olefins based on ethylene conversion ~88 %; mass yield to aromatics based on ethylene conversion ~4 %.

### Example 8

Two-Stage reactor configuration with removal of water from ethanol dehydration: Reaction Conditions: T=360 °C in first reactor, T=380 °C in second reactor WHSV = 2.1 (ethanol basis), P=0 bar; Catalysts - zirconated (5.6 wt. %) γ-alumina in first reactor and boron doped ZSM-5 zeolite (3.5 wt. % boron) admixed with Mo-alumina (W ~15 wt. %) in second reactor.

### Reactor Effluent Composition - Wt. % of Total:

| | |
|---|---|
| Ethylene | 44 |
| Propylene | 12 |
| Butenes | 22 |
| C₅ olefins | 15 |
| C₂-C₄ saturates | 6 |
| Aromatics | 0.5 |
| C₆₊ olefins | 0.5 |

Ethylene conversion ~45 %; mass yield to C₃-C₇ olefins based on ethylene conversion ~87 %; mass yield to aromatics based on ethylene conversion ~2 %.

### Example 9

Single Stage reactor configuration: Reaction Conditions: T = 355 °C in reactor, WHSV = 2.1 (ethanol basis), P = 0 bar; Catalysts - zirconated (5.6 wt. %) γ-alumina in first catalyst bed and Mo-zirconium (Mo ~15 wt. %) admixed with ZSM-5 zeolite in second catalyst bed.

### Reactor Effluent Composition - Wt. % of Total:

| | |
|---|---|
| Ethylene | 29 |
| Propylene | 16 |
| Butenes | 21 |
| C₅ olefins | 11 |
| C₂-C₄ saturates | 9 |
| Aromatics | 10 |
| C₆₊ olefins | 4 |

Ethylene conversion ~60 %; mass yield to C₃-C₇ olefins based on ethylene conversion ~75 %; mass yield to aromatics based on ethylene conversion ~18 %.

### Example 10

Reaction Conditions: T=350 °C, WHSV = 2.3; First Catalyst Bed-zirconated γ-alumina (2.9g); Second catalyst bed- tungstated zirconium (1.7g) admixed with ZSM5 zeolite (0.3g); Feed Fuel Grade Ethanol (1% water)

### Reactor Effluent Composition - Wt%:

| | | |
|---|---|---|
| Vapor phase | | 78% |
| | • Mixed Butenes | 39% |
| | • Propylene | 33% |
| | • Ethylene | 18% |
| | • C₅ olefins | 9% |
| | • Other 'lights' | 1% |

| | | |
|---|---|---|
| Organic liquid phase | | 22% |
| | • Toluene | 22% |
| | • Ethyl Benzene | 6% |
| • Xylenes | | 32% |
| • C9 Aromatics | | 17% |
| | Others | 23% |

### Example 11

Reaction Conditions: T=330 °C, WHSV = 2.3; First Catalyst Bed - zirconated γ-alumina (2.9g); Second catalyst bed-tungstated zirconium (1.7g) admixed with ZSM5 zeolite (0.3g); Feed Fuel Grade Ethanol (1% water)

### Reactor Effluent Composition - Wt%:

| | | |
|---|---|---|
| Vapor phase | | 90% |
| | • Mixed Butenes | 26% |
| | • Propylene | 18% |
| | • Ethylene | 44% |
| | • C₅ olefins | 11% |
| | • Other 'lights' | 1% |

| | | |
|---|---|---|
| Organic liquid phase | | 10% |
| | • Toluene | 10% |
| | • Ethyl Benzene | 4% |
| | • Xylenes | 26% |
| | • C9 Aromatics | 31% |
| | • Others | 29% |

### Example 12

Two-Stage reactor wherein the first reactor comprises a first catalyst: zirconated γ-alumina (2.9g); Fuel Grade Ethanol feed rate = 0.14 ml/min; T=360 °C; P=atm; the second reactor comprises a second catalyst: boron doped ZSM5 Zeolite (2.5g); Water feed = 0.02 ml/min; T=400 °C; P=atm.

### Reactor Effluent Composition - Wt. % of Total:

| | |
|---|---|
| Vapor Phase | |
| Ethylene | 31.61 |
| Propylene | 19.61 |
| Butenes | 25.37 |
| C₅ olefins | 15.95 |
| Ethane | 0.99 |
| Propane | 2.36 |
| Butane | 4.11 |

### Prophetic Example 13

Two-Stage reactor configuration without removal of water from ethanol dehydration: Reaction Conditions: T=360 °C in first reactor, T=400 °C in second reactor WHSV = 2.1 (ethanol basis), P=1 bar; Catalysts - zirconated (4 wt. %) γ-Alumina in first reactor and boron/phosphorus doped ZSM-5 zeolite (4 wt. % boron/2 wt. % phosphorus) admixed with W-SiO₂ (W ~15 wt. %) in second reactor.

### Reactor Effluent Composition - Wt. % of Total:

| | |
|---|---|
| ethylene | 39 |
| Propylene | 17 |
| Butenes | 24 |
| C₅ olefins | 14 |
| C₂-C₄ saturates | 4 |
| Aromatics | 1 |
| C₆₊ olefins | 1 |

Ethylene conversion ~50 %; mass yield to C₃-C₇ olefins based on ethylene conversion ~92 %; mass yield to aromatics based on ethylene conversion ~3 %.

### Prophetic Example 14

Two-Stage reactor configuration with removal of water from ethanol dehydration: Reaction Conditions: T=360 °C in first reactor, T=370 °C in second reactor WHSV = 2.1 (ethanol basis), P=1 bar; Catalysts - zirconated (4 wt. %) γ-alumina in first reactor and boron doped ZSM-5 zeolite (4 wt. % boron) admixed with W-zirconium (W ~15 wt. %) in second reactor.

### Reactor Effluent Composition - Wt. % of Total:

| | |
|---|---|
| Ethylene | 32 |
| Propylene | 13 |
| Butenes | 26 |
| C₅ olefins | 18 |
| C₂-C₄ saturates | 6 |
| Aromatics | 3 |
| C₆₊ olefins | 2 |

Ethylene conversion ~65 %; mass yield to C₃-C₇ olefins based on ethylene conversion ~89 %; mass yield to aromatics based on ethylene conversion ~4 %.

### Prophetic Example 15

Single Stage reactor configuration: Reaction Conditions: T=355 °C in reactor, WHSV = 2.1 (ethanol basis), P=1 bar; Catalysts -zirconated (5.6 wt. %) γ-alumina in first catalyst bed and Mo-zirconium (Mo ~15 wt. %) admixed with ZSM-5 zeolite in second catalyst bed.

### Reactor Effluent Composition - Wt. % of Total:

| | |
|---|---|
| Ethylene | 29 |
| Propylene | 16 |
| Butenes | 21 |
| C₅ olefins | 11 |
| C₂-C₄ saturates | 9 |
| Aromatics | 10 |
| C₆₊ olefins | 4 |

Ethylene conversion ~60 %; mass yield to C₃-C₇ olefins based on ethylene conversion ~80 %; mass yield to aromatics based on ethylene conversion ~15 %.

The preceding specific examples are intended to be illustrative of the invention and should not be construed as limiting the scope of the invention as defined by appended claims.

## Claims

1. A process for converting one or more C₂-C₅ linear or branched alcohols to one or more C₂-C₇ olefins, the process comprising:
contacting a first stream comprising the C₂-C₅ linear or branched alcohols with a first catalyst in a first reactor at a temperature from 200 °C to 500 °C, gauge pressure from 0 to 30 bar, and a WHSV of at least 0.5, wherein the first catalyst comprises an alumina catalyst including one or more of Si, Zr, Ti, Nb, W or F in neutral or ionic form, to form a second stream; and
contacting the second stream with a second catalyst in a second reactor at a temperature from 200 °C to 500 °C, gauge pressure from 0 to 30 bar, and a WHSV of at least 0.5, wherein the second catalyst comprises a doped or undoped zeolite catalyst and/or a doped or undoped W-Zr catalyst and forming the one or more C₂-C₇ olefins in a mass yield of at least 65 %.

2. The process of claim 1, wherein the one or more C₂-C₅ linear or branched alcohols are bio-based and produced by fermentative processes.

3. The process of claim 1, wherein the one or more C₂-C₅ linear or branched alcohols are not derived from petroleum.

4. The process of any one of claims 1 to 3, wherein the one or more C₂-C₇ olefins includes ethylene, propylene, butenes, C₅-C₆ olefins, or a combination thereof.

5. The process of any one of claims 1 to 4, wherein the temperature of the first reactor is from 300 °C to 420 °C.

6. The process of any one of claims 1 to 5, wherein the gauge pressure of the first reactor and second reactor are each independently from 0 to 10 bar.

7. The process of any one of claims 1 to 6, wherein the WHSV of the first and second reactors are independently from 0.5 to 10.

8. The process of any one of claims 1 to 7, wherein the first catalyst comprises γ-alumina catalyst.

9. The process of claim 8, wherein the γ-alumina catalyst includes Zr.

10. The process of any one of claims 1 to 9, wherein the first catalyst does not include a zeolite catalyst.

11. The process of any one of claims 1 to 10, wherein the second catalyst comprises a doped ZSM-5 zeolite catalyst.

12. The process of claim 11, wherein the doped ZSM-5 zeolite catalyst includes boron, phosphorus, or both.

13. The process of any one of claims 10 to 12, wherein the second catalyst further comprises γ-alumina catalyst, W-SiO₂ catalyst, or W-Zr catalyst, and wherein the γ-alumina optionally includes Zr, W, or Mo.

14. The process of any one of claims 1 to 13, further comprising separating a portion of water from the second stream.

15. The process of any one of claims 1 to 14, wherein the second stream comprises water and lower carbon chain olefins.

## Patentansprüche

1. Verfahren zur Umwandlung eines oder mehrerer linearer oder verzweigter C₂-C₅-Alkohole in ein oder mehrere C₂-C₇-Olefine, wobei das Verfahren umfasst:
Inkontaktbringen eines ersten Stroms, der die linearen oder verzweigten C₂-C₅-Alkohole enthält, mit einem ersten Katalysator in einem ersten Reaktor bei einer Temperatur zwischen 200 °C und 500 °C, einem Manometerdruck zwischen 0 und 30 bar und einer WHSV von wenigstens 0,5, wobei der erste Katalysator einen Aluminiumoxidkatalysator umfasst, der eines oder mehrere der Elemente Si, Zr, Ti, Nb, W oder F in neutraler oder ionischer Form enthält, um einen zweiten Strom zu bilden; und
Inkontaktbringen des zweiten Stroms mit einem zweiten Katalysator in einem zweiten Reaktor bei einer Temperatur zwischen 200 °C und 500 °C, einem Manometerdruck zwischen 0 und 30 bar und einer WHSV von wenigstens 0,5, wobei der zweite Katalysator einen dotierten oder undotierten Zeolithkatalysator und/oder einen dotierten oder undotierten W-Zr-Katalysator umfasst, und Bilden des einen oder der mehreren C₂-C₇-Olefine in einer Massenausbeute von wenigstens 65 %.

2. Verfahren nach Anspruch 1, wobei der eine oder die mehreren linearen oder verzweigten C₂-C₅-Alkohole biobasiert sind und durch fermentative Verfahren hergestellt werden.

3. Verfahren nach Anspruch 1, wobei der eine oder die mehreren linearen oder verzweigten C₂-C₅-Alkohole nicht aus Erdöl stammen.

4. Verfahren nach einem der Ansprüche 1 bis 3, wobei das eine oder die mehreren C₂-C₇-Olefine Ethylen, Propylen, Butene, C₅-C₆-Olefine oder eine Kombination davon umfassen.

5. Verfahren nach einem der Ansprüche 1 bis 4, wobei die Temperatur des ersten Reaktors zwischen 300 °C und 420 °C beträgt.

6. Verfahren nach einem der Ansprüche 1 bis 5, wobei der Manometerdruck des ersten Reaktors und des zweiten Reaktors unabhängig voneinander jeweils zwischen 0 und 10 bar beträgt.

7. Verfahren nach einem der Ansprüche 1 bis 6, wobei die WHSV des ersten und des zweiten Reaktors unabhängig voneinander jeweils zwischen 0,5 und 10 beträgt.

8. Verfahren nach einem der Ansprüche 1 bis 7, wobei der erste Katalysator einen γ-Aluminiumoxidkatalysator umfasst.

9. Verfahren nach Anspruch 8, wobei der γ-Aluminiumoxidkatalysator Zr enthält.

10. Verfahren nach einem der Ansprüche 1 bis 9, wobei der erste Katalysator keinen Zeolithkatalysator umfasst.

11. Verfahren nach einem der Ansprüche 1 bis 10, wobei der zweite Katalysator einen dotierten ZSM-5-Zeolithkatalysator umfasst.

12. Verfahren nach Anspruch 11, wobei der dotierte ZSM-5-Zeolithkatalysator Bor, Phosphor oder beides enthält.

13. Verfahren nach einem der Ansprüche 10 bis 12, wobei der zweite Katalysator ferner einen γ-Aluminiumoxidkatalysator, W-SiO₂-Katalysator oder W-Zr-Katalysator umfasst und wobei das γ-Aluminiumoxid optional Zr, W oder Mo enthält.

14. Verfahren nach einem der Ansprüche 1 bis 13, das ferner das Abscheiden eines Teils des Wassers aus dem zweiten Strom umfasst.

15. Verfahren nach einem der Ansprüche 1 bis 14, wobei der zweite Strom Wasser und Olefine mit kürzerer Kohlenstoffkette umfasst.

## Revendications

1. Procédé pour convertir un ou plusieurs alcools linéaires ou ramifiés en C₂-C₅ en une ou plusieurs oléfines en C₂-C₇, le procédé comprenant :
la mise en contact d'un premier flux comprenant les alcools linéaires ou ramifiés en C₂-C₅ avec un premier catalyseur dans un premier réacteur à une température de 200 °C à 500 °C, une pression manométrique de 0 à 30 bars et une WHSV d'au moins 0,5, dans lequel le premier catalyseur comprend un catalyseur à base d'alumine comprenant un ou plusieurs éléments parmi le Si, le Zr, le Ti, le Nb, le W ou le F sous forme neutre ou ionique, pour former un second flux ; et
la mise en contact du second flux avec un second catalyseur dans un second réacteur à une température de 200 °C à 500 °C, une pression manométrique de 0 à 30 bars, et une WHSV d'au moins 0,5, dans lequel le second catalyseur comprend un catalyseur à base de zéolite dopée ou non dopée et/ou un catalyseur à base de W-Zr dopé ou non dopé, et la formation des une ou plusieurs oléfines en C₂-C₇ avec un rendement massique d'au moins 65 %.

2. Procédé selon la revendication 1, dans lequel les un ou plusieurs alcools linéaires ou ramifiés en C₂-C₅ sont biosourcés et produits par des procédés de fermentation.

3. Procédé selon la revendication 1, dans lequel les un ou plusieurs alcools linéaires ou ramifiés en C₂-C₅ ne sont pas dérivés du pétrole.

4. Procédé selon l'une quelconque des revendications 1 à 3, dans lequel les une ou plusieurs oléfines en C₂-C₇ comprennent l'éthylène, le propylène, les butènes, les oléfines en C₅-C₆ ou une combinaison de ceux-ci.

5. Procédé selon l'une quelconque des revendications 1 à 4, dans lequel la température du premier réacteur est de 300 °C à 420 °C.

6. Procédé selon l'une quelconque des revendications 1 à 5, dans lequel la pression manométrique du premier réacteur et celle du second réacteur sont chacune, indépendamment, comprises entre 0 et 10 bars.

7. Procédé selon l'une quelconque des revendications 1 à 6, dans lequel la WHSV des premier et second réacteurs est indépendamment de 0,5 à 10.

8. Procédé selon l'une quelconque des revendications 1 à 7, dans lequel le premier catalyseur comprend un catalyseur à base de γ-alumine.

9. Procédé selon la revendication 8, dans lequel le catalyseur à base de γ-alumine comprend du Zr.

10. Procédé selon l'une quelconque des revendications 1 à 9, dans lequel le premier catalyseur ne comprend pas de catalyseur à base de zéolite.

11. Procédé selon l'une quelconque des revendications 1 à 10, dans lequel le second catalyseur comprend un catalyseur à base de zéolite ZSM-5 dopée.

12. Procédé selon la revendication 11, dans lequel le catalyseur à base de zéolite ZSM-5 dopée comprend du bore, du phosphore ou les deux.

13. Procédé selon l'une quelconque des revendications 10 à 12, dans lequel le second catalyseur comprend en outre un catalyseur à base de γ-alumine, un catalyseur à base de W-SiO₂ ou un catalyseur à base de W-Zr, et dans lequel l'γ-alumine comprend éventuellement du Zr, du W ou du Mo.

14. Procédé selon l'une quelconque des revendications 1 à 13, comprenant en outre la séparation d'une partie de l'eau du second flux.

15. Procédé selon l'une quelconque des revendications 1 à 14, dans lequel le second flux comprend de l'eau et des oléfines à chaîne carbonée courte.
